# EUROPÄISCHE PATENTSCHRIFT

(11) **EP 0 745 581 B1**
(45) Veröffentlichungstag und Bekanntmachung des Hinweises auf die Patenterteilung: **05.04.2000**
(21) Anmeldenummer: 96108081.9
(22) Anmeldetag: 21.05.1996
(51) Int. Cl.: C07C 229/16, C07C 227/26, C07C 227/24, C07C 227/14, C07C 227/16

(54) **Verfahren zur Herstellung von Glycin-N,N-diessigsäure-Derivaten durch Umsetzung von Glycinderivaten oder deren Vorstufen mit Formaldehyd und Alkalimetallcyanid in wässrig-alkalischem Medium**
Process for the preparation of glycine-N,N-diacetic acid derivatives from the reaction of glycine derivatives or their precursors with formaldehyde and an alkalimetal cyanide in an acidic aqueous medium
Procédé pour la préparation de dérivés de glycine-N,N-diacétique par réaction de dérivés de la glycine ou de leurs précurseurs avec le formaldéhyde et du cyanure de métal alcalin dans un milieu acide aqueux

(30) Priorität: 29.05.1995 DE 19518987
(43) Veröffentlichungstag der Anmeldung: 04.12.1996
(73) Patentinhaber: BASF Aktiengesellschaft, 67063 Ludwigshafen (DE)
(72) Erfinder: Greindl, Thomas, Dr., 94127 Neuburg (DE); Oftring, Alfred, Dr., 67098 Bad Dürkheim (DE); Braun, Gerold, Dr., 67071 Ludwigshafen (DE); Wild, Jochen, Dr., 67152 Ruppertsberg (DE); Potthoff-Karl, Birgit, Dr., 67061 Ludwigshafen (DE); Schuh, Georg, 67067 Ludwigshafen (DE)

(56) Entgegenhaltungen:
- EP-A- 0 287 885
- DE-A- 2 027 972
- DE-A- 4 319 935
- US-A- 2 500 019
- US-A- 3 733 355

## Beschreibung

Die vorliegende Erfindung betrifft ein Verfahren zur Herstellung von Glycin-N,N-diessigsäure-Derivaten durch Umsetzung von Glycinderivaten oder deren Vorstufen mit Formaldehyd und Alkalimetallcyanid in wäßrig-alkalischem Medium.

In den typischen Anwendungsgebieten von Komplexbildern und Buildern, wie hochalkalische Reiniger und Haushaltswaschmittel, werden heute als Standardprodukte Aminopolyphosphonate, Polycarboxylate oder Ethylendiamintetraessigsäure (EDTA) eingesetzt. Diese Produkte sind nur schwer biologisch abbaubar, deshalb besteht ein Bedarf nach wirksamen und gleichzeitig billigen biologisch leicht abbaubaren Substituten.

Eine Alternative zu obigen Stoffen stellt Nitrilotriessigsäure (NTA) dar, sie ist leicht biologisch abbaubar, hat aber deutliche Wirkungsnachteile gegenüber EDTA und ist aus toxikologischen Gründen häufig unerwünscht. Methylglycin-N,N-diessigsäure (α-Alanin-N,N-diessigsäure, MGDA) ist ein nicht toxischer, leicht biologisch abbaubarer Komplexbildner mit höherer Komplexbildungskonstante als NTA. Die Verwendung von MGDA und verwandter Glycin-N,N-diessigsäure-Derivate für den Wasch- und Reinigungsmittelsektor und für zahlreiche neue Anwendungen sowie neue Syntheserouten für solche Substanzen werden in der WO-A 94/29421 (1) beschrieben.

Die Synthese von MGDA mit Hilfe von Chloressigsäure ist seit langem bekannt. Dieser Weg ist heute aufgrund des Zwangsanfalls von Natriumchlorid und der Bildung chlororganischer Verunreinigungen nicht mehr wirtschaftlich und vom ökologischen Standpunkt her auch nicht mehr zeitgemäß. Auch muß, um hohe Ausbeuten zu erreichen, Chloressigsäure im Überschuß eingesetzt werden, dabei kommt es zur Bildung von Glykolsäure, Oxodiacetat und chlororganischen Verbindungen als Nebenprodukten. Andere Halogenessigsäuren bilden ein ähnliches Nebenproduktspektrum aus. Die Abtrennung der stöchiometrisch anfallenden anorganischen Salze wie Natriumchlorid ist aufwendig und kostspielig.

Eine wirtschaftliche und gleichzeitig umweltschonende Methode zur Herstellung von Aminopolycarboxylaten ist prinzipiell die Strekker-Reaktion von Aminosäuren. Die Synthese von MGDA mit Hilfe der Strecker-Reaktion wird in (1) beschrieben.

In der DE-A 20 27 972 (2) wird die "saure" Variante der Strecker-Reaktion von unsubstituiertem Glycin mit Formaldehyd und Blausäure beschrieben. Hierbei bildet sich aus Glycin das N,N-Bis(cyanmethyl)glycin, welches in hoher Reinheit isoliert werden kann. Der Nachteil der "sauren" Variante liegt im Umgang mit freier Blausäure und in der Notwendigkeit eines zusätzlichen Verseifungsschrittes nach Abtrennung des Nitrils.

Die "alkalische" Variante der Strecker-Reaktion wird beispielsweise in der US-A 3 733 355 (3) in allgemeiner Form dargestellt. Die dort aufgeführten Beispiele zeigen jedoch, daß immer ein hoher Anteil an Nebenprodukten, vor allem an unerwünschter Glykolsäure, auftritt; dies läßt sich aus den Umsätzen von nur maximal ca. 89 % schließen.

In der US-A 2 500 019 (4) wird die Umsetzung von α-Aminosäuren mit Formaldehyd und Natriumcyanid allgemein erwähnt und am Beispiel von unsubstituiertem Glycin aus Proteinhydrolysat Nitrilotriessigsäure hergestellt. Bei diesem Verfahren wird allerdings mit Glycin eine besonders reaktive, weil unsubstituierte Aminosäure eingesetzt. Das sich bildende NTA ist zudem aufgrund seiner hohen Symmetrie thermodynamisch gegenüber unsymmetrischen Verbindungen bevorzugt und bildet sich besonders leicht.

Gerade die Umsetzung von stärker sterisch gehinderten Aminosäuren, wie etwa Alanin, in hohen Ausbeuten mit möglichst geringen Anteilen an NTA ist schwierig. In (4) wird Glycin-Natrium-Salz aus Proteinhydrolysat hergestellt. Ein Proteinhydrolysat enthält in der Regel Beimengungen von anderen Aminosäuren, so daß die Umsetzung nach Strecker hier zu keinem NTA-reinen Produkt führt.

Aufgabe der vorliegenden Erfindung war daher die Bereitstellung einer einfachen und wirtschaftlichen Syntheseroute für Glycin-N,N-diessigsäuren wie MGDA, dabei sollte eine möglichst hohe Gesamtausbeute bei gleichzeitig hohen Produktreinheiten, mit niedrigen NTA-Gehalten, möglichst unter 2 Gew.-%, angestrebt werden.

Demgemäß wurde ein Verfahren zur Herstellung von Glycin-N,N-diessigsäure-Derivaten der allgemeinen Formel I 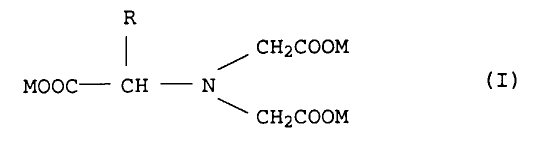 in der
- R: für C₁- bis C₃₀-Alkyl oder C₂- bis C₃₀-Alkenyl, welche zusätzlich als Substituenten bis zu 5 Hydroxylgruppen, Formylgruppen, C₁- bis C₄-Alkoxygruppen, Phenoxygruppen oder C₁- bis C₄-Alkoxycarbonylgruppen tragen und durch bis zu 5 nicht benachbarte Sauerstoffatome unterbrochen sein können, Alkoxylat-Gruppierungen der Formel -(CH₂)ₖ-O-(A¹O)ₘ-(A²O)ₙ-Y, in der A¹ und A² unabhängig voneinander 1,2-Alkylengruppen mit 2 bis 4 C-Atomen bezeichnen, Y Wasserstoff, C₁- bis C₁₂-Alkyl, Phenyl oder C₁- bis C₄-Alkoxycarbonyl bedeutet und k für die Zahl 1, 2 oder 3 sowie m und n jeweils für Zahlen von 0 bis 50 stehen, wobei die Summe aus m + n mindestens 4 betragen muß, Phenylalkylgruppen mit 1 bis 20 C-Atomen im Alkyl, Phenyl, einen fünf- oder sechsgliedrigen ungesättigten oder gesättigten heterocyclischen Ring mit bis zu drei Heteroatomen aus der Gruppe Stickstoff, Sauerstoff und Schwefel, welcher zusätzlich benzanelliert sein kann, wobei alle bei den Bedeutungen für R genannten Phenylkerne und heterocyclischen Ringe noch zusätzlich als Substituenten bis zu drei C₁- bis C₄-Alkylgruppen, Hydroxylgruppen, Carboxylgruppen, Sulfogruppen oder C₁- bis C₄-Alkoxycarbonylgruppen tragen können, oder einen Rest der Formel 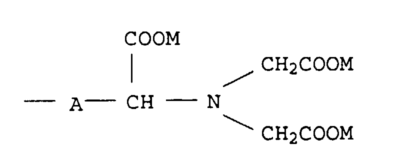 steht, wobei A eine C₁- bis C₁₂-Alkylen-Brücke oder eine chemische Bindung bezeichnet, und
- M: Wasserstoff, Alkalimetall, Erdalkalimetall-, Ammonium oder substituiertes Ammonium in den entsprechenden stöchiometrischen Mengen bedeutet,
durch Umsetzung von entsprechenden 2-substituierten Glycinen oder 2-substituierten Glycinnitrilen oder verdoppelten Glycinen der Formel 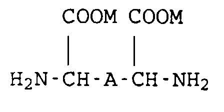 oder verdoppelten Glycinnitrilen der Formel 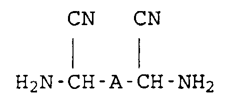 oder von Alaninaminonitril oder 5-Methylhydantoin im Falle von Alanin als Vorstufen der als Ausgangsmaterial genannten Glycinderivate mit Formaldehyd und Alkalimetallcyanid in wäßrigem Medium bei einem pH-Wert von 8 bis 14 gefunden, welches dadurch gekennzeichnet ist, daß man das Ausgangsmaterial im wäßrigen Reaktionsmedium auf Umsetzungstemperatur bringt, danach 0,5 bis 30 % der zur Umsetzung benötigten Menge an Alkalimetallcyanid zu den vorgelegten Glycinderivaten oder deren Vorstufen gibt und anschließend die restliche Menge Alkalimetallcyanid und den Formaldehyd gleichzeitig über einen Zeitraum von 0,5 bis 12 Stunden zudosiert.

Bei den aus dem Stand der Technik bekannten Durchführungsformen der "alkalischen" Strecker-Reaktion aus Aminosäuren werden meist entweder die Gesamtmengen an Formaldehyd und Alkalimetallcyanid vor Beginn der Umsetzung auf einmal zugesetzt oder die Gesamtmenge an Alkalimetallcyanid wird zugegeben und danach der Formaldehyd zudosiert.

Das erfindungsgemäße Verfahren unterscheidet sich demgegenüber dadurch, daß die als Ausgangsmaterial vorgelegten Glycinderivate oder deren Vorstufen im wäßrigen Reaktionsmedium auf Umsetzungstemperatur gebracht und danach 0,5 bis 30 %, vorzugsweise 1,0 bis 15 %, insbesondere 2,0 bis 10 % der zur Umsetzung benötigten Menge an Alkalimetallcyanid auf einmal zugegeben werden. Anschließend werden die restliche Menge Alkalimetallcyanid und der Formaldehyd gleichzeitig über einen Zeitraum von 0,5 bis 12, vorzugsweise 1 bis 8, insbesondere 2 bis 6 Stunden zudosiert. Dabei können die Zugabeenden von Alkalimetallcyanid und Formaldehyd gleichzeitig oder zeitlich versetzt liegen, wobei im zweiten Fall die Formaldehyd-Zugabe meist später beendet ist. Im Anschluß an die Zugabe der Umsetzungskomponenten läßt man üblicherweise noch 1 1 bis 10, vorzugsweise 2 bis 5 Stunden unter den Umsetzungsbedingungen nachreagieren.

Alkalimetallcyanid, in der Regel Natrium- oder Kaliumcyanid, und Formaldehyd werden normalerweise als wäßrige Lösungen eingesetzt. Man kann aber auch beispielsweise diese Komponenten in fester Form (im Falle des Formaldehyds z.B. als Paraformaldehyd) zugeben. Als Umsetzungsmedium dient in der Regel Wasser, das die Endprodukte sowie die eingesetzten Umsetzungskomponenten meist in ausreichendem Maße löst. Man kann aber auch Mischungen aus Wasser und wassermischbaren organischen Lösungsmitteln wie Alkoholen, z.B. Methanol, Ethanol oder Isopropanol, verwenden, wenn beispielsweise Glycin-N,N-diessigsäuren I mit einem hydrophoberen, d.h. längerkettigen oder voluminöseren Rest R hergestellt werden sollen.

In einer bevorzugten Ausführungsform des erfindungsgemäßen Verfahrens legt man vor und/oder während der Umsetzung ein Vakuum von 10 bis 100 kPa (100 bis 1000 mbar), vorzugsweise 30 bis 90 kPa (300 bis 900 mbar), insbesondere 50 bis 80 kPa (500 bis 800 mbar) an die Reaktionsapparatur an.

In einer weiteren bevorzugten Ausführungsform des erfindungsgemäßen Verfahrens leitet man vor und/oder während der Umsetzung ein Inertgas wie Luft, Stickstoff oder Argon durch die Reaktionsmischung bzw. die vorgelegten Umsetzungskomponenten ("Strippung" mit Inertgas).

Man kann beide genannten bevorzugten Ausführungsformen auch kombinieren. Das Vakuum und das Durchleiten von Inertgas dienen insbesondere dazu, noch aus den Vorstufen enthaltenden bzw. während der Umsetzung sich bildenden Ammoniak aus dem Reaktionssystem besser zu entfernen. Es hat sich auch gezeigt, daß die Umsetzung bei Normaldruck oder die Anwendung eines leichten Überdruckes zu unerwünscht hohen Gehalten an NTA (meist > 2 Gew.-%) im Endprodukt führt.

Die erfindungsgemäße Umsetzung der eingesetzten Glycinderivate oder deren Vorstufen mit Formaldehyd und Alkalimetallcyanid wird üblicherweise bei Temperaturen von 40 bis 110°C, insbesondere 60 bis 100°C, vor allem 75 bis 90°C durchgeführt. Der pH-Wert des wäßrigen Umsetzungsmediums liegt bei 8 bis 14, vorzugsweise 10 bis 13.

Man setzt pro Mol als Ausgangsmaterial verwendetem Glycinderivat oder dessen Vorstufe zweckmäßigerweise 2,0 bis 3,0 mol, insbesondere 2,0 bis 2,6 mol Formaldehyd, vorzugsweise in Form seiner wäßrigen ca. 30 gew.-%igen Lösung, und insgesamt 2,0 bis 3,0 mol, insbesondere 2,0 bis 2,6 mol Alkalimetallcyanid, vorzugsweise als wäßrige ca. 20 bis 40 gew.-%ige Lösung, ein. Üblicherweise verwendet man als Ausgangsmaterial wäßrige Lösungen der entsprechenden Glycinderivate bzw. Vorstufen mit einem Glycinderivat- bzw. Vorstufen-Gehalt von 10 bis 50 Gew.-%, insbesondere 25 bis 45 Gew.-%.

Das erfindungsgemäße Verfahren liefert überraschenderweise auch hervorragende Ergebnisse, wenn man als Ausgangsmaterial aus der technischen Synthese von Glycinderivaten oder Alaninaminonitril oder 5-Methylhydantoin im Falle von Alanin als deren Vorstufen stammendes nicht gereinigtes, d.h. in der Regel nicht als Feststoff isoliertes oder z.B. durch Kristallisation von Nebenbestandteilen befreites Rohmaterial oder bei solchen Synthesen anfallende Mutterlaugen einsetzt.

Unter Vorstufen von Glycinderivaten sind im Falle von Alanin (R=CH₃) Alaninaminonitril oder 5-Methylhydantoin zu verstehen, wobei letzteres z.B. durch Umsetzung von Acetaldehyd, Alkalimetallcyanid und Ammoniumcarbonat entsteht. Die übliche technische Alanin-Synthese wird nach Strecker durch Umsetzung von Acetaldehyd, Blausäure und Ammoniak durchgeführt. Auch enzymatisch hergestelltes Alanin kann ohne Feststoffisolierung eingesetzt werden.

Mit besonders guten Ergebnissen kann das erfindungsgemäße Verfahren zur Herstellung von Glycin-N,N-diessigsäure-Derivaten I angewandt werden, bei denen R für C₁- bis C₂₀-Alkyl, C₂- bis C₂₀-Alkenyl oder einen Rest der Formel 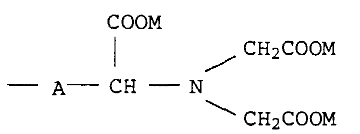 steht.

Ganz besonders gut eignet sich das erfindungsgemäße Verfahren zur Herstellung von α-Alanin-N,N-diessigsäure (R=CH₃) und ihren Alkalimetall-, Ammonium- und substituierten Ammoniumsalzen.

Als derartige Salze kommen vor allem die Natrium-, Kalium- und Ammoniumsalze, insbesondere das Trinatrium-, Trikalium- und Triammoniumsalz, sowie organische Triaminsalze mit einem tertiären Stickstoffatom in Frage.

Als den organischen Aminsalzen zugrundeliegende Basen kommen insbesondere tertiäre Amine wie Trialkylamine mit 1 bis 4 C-Atomen im Alkyl, z.B. Trimethyl- und Triethylamin, und Trialkanolamine mit 2 oder 3 C-Atomen im Alkanolrest, bevorzugt Triethanolamin, Tri-n-propanolamin oder Triisopropanolamin, in Betracht.

Als Erdalkalimetallsalze werden insbesondere die Calcium- und Magnesiumsalze eingesetzt.

Neben Methyl kommen für den Rest R als geradkettige oder verzweigte Alk(en)ylreste vor allem C₂- bis C₁₇-Alkyl und -Alkenyl, hierbei insbesondere geradkettige, von gesättigten oder ungesättigten Fettsäuren abgeleitete Reste, in Betracht. Beispiele für einzelne Reste R sind: Ethyl, n-Propyl, iso-Propyl, n-Butyl, iso-Butyl, sec.-Butyl, tert.-Butyl, n-Pentyl, iso-Pentyl, sec.-Pentyl, tert.-Pentyl, Neopentyl, n-Hexyl, n-Heptyl, 3-Heptyl (abgeleitet von 2-Ethylhexansäure), n-Octyl, iso-Octyl (abgeleitet von iso-Nonansäure), n-Nonyl, n-Decyl, n-Undecyl, n-Dodecyl, iso-Dodecyl (abgeleitet von iso-Tridecansäure), n-Tridecyl, n-Tetradecyl, n-Pentadecyl, n-Hexadecyl, n-Heptadecyl, n-Octadecyl, n-Nonadecyl, n-Eicosyl und n-Heptadecenyl (abgeleitet von Ölsäure). Es können für R auch Gemische auftreten, insbesondere solche, die sich von natürlich vorkommenden Fettsäuren und von synthetisch erzeugten technischen Säuren, beispielsweise durch Oxosynthese, ableiten.

Als Beispiele für die weiterhin genannten C₁- bis C₄-, C₁- bis C₁₂- und C₁- bis C₂₀-Alkylgruppen sind auch die entsprechenden oben aufgeführten Reste für R zu verstehen.

Als C₁- bis C₁₂-Alkylen-Brücken A dienen vor allem Polymethylengruppierungen der Formel -(CH₂)ₖ-, worin k eine Zahl von 2 bis 12, insbesondere von 2 bis 8 bezeichnet, d. h. 1,2-Ethylen, 1,3-Propylen, 1,4-Butylen, Pentamethylen, Hexamethylen, Heptamethylen, Octamethylen, Nonamethylen, Decamethylen, Undecamethylen und Dodecamethylen. Hexamethylen, Octamethylen, 1,2-Ethylen und 1,4-Butylen werden hierbei besonders bevorzugt. Daneben können aber auch verzweigte C₁- bis C₁₂-Alkylengruppen auftreten, z. B. -CH₂CH(CH₃)CH₂-, -CH₂C(CH₃)₂CH₂-, -CH₂CH(C₂H₅)- oder -CH₂CH(CH₃)-.

Die C₁- bis C₃₀-Alkyl- und C₂- bis C₃₀-Alkenylgruppen können bis zu 5, insbesondere bis zu 3 zusätzliche Substituenten der genannten Art tragen und durch bis zu 5, insbesondere bis zu 3 nicht benachbarte Sauerstoffatome unterbrochen sein. Beispiele für solche substituierte Alk(en)ylgruppen sind -CH₂OH, -CH₂CH₂OH, -CH₂CH₂-O-CH₃, -CH₂CH₂-O-CH₂CH₂-O-CH₃, -CH₂-O-CH₂CH₃, -CH₂-O-CH₂CH₂-OH, -CH₂-CHO, -CH₂-OPh, -CH₂-COOCH₃ oder -CH₂CH₂-COOCH₃.

Als Alkoxylat-Gruppierungen kommen insbesondere solche in Betracht, bei denen m und n jeweils für Zahlen von 0 bis 30, vor allem von 0 bis 15 stehen. A¹ und A² bedeuten von Butylenoxid und vor allem von Propylenoxid und von Ethylenoxid abgeleitete Gruppen. Von besonderem Interesse sind reine Ethoxylate und reine Propoxylate, aber auch Ethylenoxid-Propylenoxid-Blockstrukturen können auftreten.

Als fünf- oder sechsgliedrige ungesättigte oder gesättigte heterocyclische Ringe mit bis zu drei Heteroatomen aus der Gruppe Stickstoff, Sauerstoff und Schwefel, welche zusätzlich benzanelliert und durch die bezeichneten Reste substituiert sein können, kommen in Betracht:

Tetrahydrofuran, Furan, Tetrahydrothiophen, Thiophen, 2,5-Dimethylthiophen, Pyrrolidin, Pyrrolin, Pyrrol, Isoxazol, Oxazol, Thiazol, Pyrazol, Imidazolin, Imidazol, 1,2,3-Triazolidin, 1,2,3- und 1,2,4-Triazol, 1,2,3- , 1,2,4- und 1,2,5-Oxadiazol, Tetrahydropyran, Dihydropyran, 2H- und 4H-Pyran, Piperidin, 1,3- und 1,4-Dioxan, Morpholin, Pyrazan, Pyridin, α-, β- und γ-Picolin, α- und γ-Piperidon, Pyrimidin, Pyridazin, Pyrazin, 1,2,5-Oxathiazin, 1,3,5-, 1,2,3- und 1,2,4-Triazin, Benzofuran, Thionaphthen, Indolin, Indol, Isoindolin, Benzoxazol, Indazol, Benzimidazol, Chroman, Isochroman, 2H- und 4H-Chromen, Chinolin, Isochinolin, 1,2,3,4-Tetrahydroisochinolin, Cinnolin, Chinazolin, Chinoxalin, Phthalazin und Benzo-1,2,3-triazin.

N-H-Gruppierungen in den genannten heterocyclischen Ringen sollten möglichst in derivatisierter Form, etwa als N-Alkyl-Gruppierung, vorliegen.

Bei Substitution an den Phenylkernen oder den heterocyclischen Ringen treten vorzugsweise zwei (gleiche oder verschiedene) oder insbesondere ein einzelner Substituent auf.

Beispiele für gegebenenfalls substituierte Phenylalkylgruppen und heterocyclische Ringe tragende Alkylgruppen für R sind Benzyl, 2-Phenylethyl, 3-Phenylpropyl, 4-Phenylbutyl, o-, m- oder p-Hydroxylbenzyl, o-, m- oder p-Carboxylbenzyl, o-, m- oder p-Sulfobenzyl, o-, m- oder p-Methoxy- oder -Ethoxycarbonylbenzyl, 2-Furylmethyl, N-Methylpiperidin-4-ylmethyl oder 2-, 3- oder 4-Pyridinylmethyl.

Bei Substitution an Phenylkernen und auch an heterocyclischen Ringen treten bevorzugt wasserlöslich machende Gruppen wie Hydroxylgruppen, Carboxylgruppen oder Sulfogruppen auf.

Die nach dem erfindungsgemäßen Verfahren hergestellten Verbindungen I können hinsichtlich des α-C-Atoms als Racemate oder als enantiomerenreine Verbindungen vorliegen, je nachdem, ob man von D,L-Glycinderivaten oder den entsprechenden D- oder L-Formen ausgeht.

Die freien Säuren der Verbindungen I können durch Ansäuern nach üblichen Methoden erhalten werden.

Beim erfindungsgemäßen Verfahren wird durch die spezielle Reaktionsführung die Bildung von unerwünschtem NTA im Produkt weitestgehend unterdrückt, die NTA-Mengen liegen deutlich unter 2 Gew.-%, meist bei 0,1 bis 0,3 Gew.-%. Die Ausbeuten sind deutlich höher als bei den aus dem Stand der Technik bekannten Verfahren, so liegt die Ausbeute im Falle der Herstellung von MGDA-Trinatriumsalz bei > 95 %, bezogen auf Alanin, gegenüber ca. 90 % nach dem aus (4) bekannten Verfahren. Beim erfindungsgemäßen Verfahren wird die Nebenreaktion der Bildung von Formiat aus Cyanid weitgehend unterdrückt, im Gegensatz zum Verfahren gemäß (4) wird die mittlere Verweilzeit von Cyanid in der alkalischen Lösung durch das erfindungsgemäße Verfahren verkürzt. Überraschenderweise ist die Bildung von Iminodiessigsäure als Nebenprodukt, im Gegensatz zu der in (1) beschriebenen Verseifung des entsprechenden Trinitrils zu MGDA-Trinatriumsalz, nicht zu beobachten.

Ein besonderer Vorteil des erfindungsgemäßen Verfahrens ist die Möglichkeit, anstelle von reinen Glycinderivaten auch entsprechende Rohgemische, wie sie z.B. bei der Aminosäure-Synthese nach Strecker, etwa von Alanin, oder auch enzymatisch entstehen, aber auch entsprechende Vorstufen wie Hydantoine einzusetzen. Diese Vorgehensweise ist besonders wirtschaftlich, da die im Anschluß an die Aminosäureherstellung normalerweise notwendige teure Produktabtrennung am isoelektrischen Punkt hier nicht notwendig ist. Somit können Reagenzien zur pH-Wert-Einstellung eingespart und Abtrennungsverluste vermieden werden, da auch die normalerweise in der Mutterlauge der Aminosäuresynthese verbleibende Aminosäure genutzt wird. Günstigerweise kann das Alkalimetallsalz aus der Aminosäuresynthese ohne weiteren Zusatz von Alkali direkt und ohne Ausbeute- und Selektivitätsverlust umgesetzt werden. Hydantoine können ebenfalls wie Nitrile in einem Ansatz durch Zugabe von entsprechender Menge Alkali verseift und unmittelbar in einem Arbeitsgang mit Formaldehyd in wäßrigem Alkalimetallcyanid in erfindungsgemäßer Weise umgesetzt werden, insgesamt liefert diese Methode gegenüber der Umsetzung von durch Fällung isolierter Aminosäure eine höhere Gesamtausbeute bei gleichzeitig einfacherem Verfahren.

### Beispiele

### Beispiel 1

### Herstellung von MGDA-Trinatriumsalz aus Alanin

42,0 kg 50 gew.-%ige wäßrige Natronlauge wurden in 84 kg Wasser gelöst. Zu dieser Lösung wurden bei Raumtemperatur 44,5 kg D,L-α-Alanin innerhalb 30 min eingetragen. Danach wurde auf 80°C erhitzt und 50 kPa (500 mbar) Unterdruck angelegt. Innerhalb von 3 h wurden 186 kg 33 gew.-%ige wäßrige NaCN-Lösung und, zeitversetzt 15 min später, ebenfalls innerhalb von 3 h 125 kg 30 gew.-%ige wäßrige Formaldehyd-Lösung eingetragen. Die Temperatur wurde während der ganzen Zeit bei 80°C gehalten und der entstehende Ammoniak durch das angelegte Vakuum entfernt. Nach weiteren 3 h bei 80°C verblieben 335 kg einer schwach gelben Lösung mit einem Eisenbindevermögen von 1,47 mmol/g, entsprechend einer Ausbeute an MGDA-Trinatriumsalz von 98,5 %. Der mittels HPLC ermittelte Gehalt an NTA betrug 0,24 Gew.-%.

### Beispiel 2

### Herstellung von MGDA-Trinatriumsalz aus Alanin-Rohgemisch

Zu 204 g 25 gew.-%igem wäßrigen Ammoniak wurden bei 0°C 27 g Blausäure zugetropft. Zu der entstandenen Lösung wurden dann innerhalb von 20 min 44 g Acetaldehyd bei 10°C getropft, nach weiteren 2 h bei 20°C betrug der Umsatz an HCN 98 %. Es wurden 204 g 21 gew.-%ige wäßrige NaOH bei dieser Temperatur zugegeben und 5 h gerührt. Danach wurde für 1 h auf 60°C und 3 h auf 95°C erhitzt, dabei wurde mit Stickstoff gestrippt, bis kein Ammoniak mehr entstand.

Zu dieser Rohalanin-Lösung wurden dann bei 80°C 7,5 g 33 gew.-%ige Natriumcyanid-Lösung gegeben und anschließend mittels einer automatischen Dosiervorrichtung gleichzeitig über 3 h und bei 800 mbar Vakuum sowie unter Einleiten von Stickstoff 200 g 30 gew.-%ige wäßrige Formaldehyd-Lösung und 290 g 33 gew.-%ige Natriumcyanid-Lösung zudosiert. Nach Ende der Zugabe wurde noch 2 h unter den angegebenen Bedingungen nachgerührt, man erhielt 812 g einer schwach gelben Lösung mit einem Eisenbindevermögen von 0,95 mmol/g, entsprechend 77 % der Theorie, bezogen auf Acetaldehyd. Der Gehalt an NTA betrug 0,27 Gew.-%.

### Beispiel 3

### Herstellung von MGDA aus 5-Methylhydantoin

106 g Ammoniumcarbonat, gelöst in 500 ml Wasser, wurden zusammen mit 149 g 33 gew.-%iger wäßriger Natriumcyanid-Lösung bei 10°C vorgelegt, dazu tropfte man innerhalb von 2 h 44 g Acetaldehyd, nach Ende der Zugabe wurde noch 24 h bei 20°C gerührt, der Umsatz an Cyanid betrug nach dieser Zeit 100 %. Zu der Mischung wurden 3,40 g Imidazol gegeben und portionsweise bei 95°C insgesamt 120 g NaOH eingetragen. Nach 64 h bei ca. 100°C bei gleichzeitiger Strippung mit Stickstoff wurden zu dieser 5-Methylhydantoin enthaltenden Lösung 8 g 33 gew.-%ige wäßrige Natriumcyanid-Lösung i gegeben. Bei 80°C und 80 kPa (800 mbar) Druck wurden unter gleichzeitiger Strippung mit Stickstoff zeitgleich 280 g 33 gew.-%ige wäßrige Natriumcyanid-Lösung sowie 200 g 30 gew.-%iger wäßriger Formaldehyd innerhalb von 3 h zugegeben. Nach weiteren 3 h unter diesen Bedingungen erhielt man 631 g einer Lösung mit einem Eisenbindevermögen von 1,28 mmol/g, entsprechend 81 % der Theorie, der NTA-Gehalt betrug 0,25 Gew.-%.

## Patentansprüche

1. Verfahren zur Herstellung von Glycin-N,N-diessigsäure-Derivaten der allgemeinen Formel I 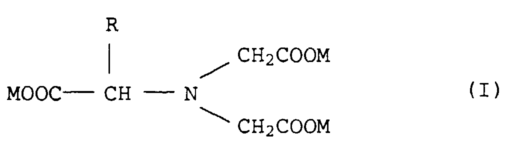 in der
R für C₁- bis C₃₀-Alkyl oder C₂- bis C₃₀-Alkenyl, welche zusätzlich als Substituenten bis zu 5 Hydroxylgruppen, Formylgruppen, C₁- bis C₄-Alkoxygruppen, Phenoxygruppen oder C₁- bis C₄-Alkoxycarbonylgruppen tragen und durch bis zu 5 nicht benachbarte Sauerstoffatome unterbrochen sein können, Alkoxylat-Gruppierungen der Formel -(CH₂)ₖ-O-(A¹O)ₘ-(A²O)ₙ-Y, in der A¹ und A² unabhängig voneinander 1,2-Alkylengruppen mit 2 bis 4 C-Atomen bezeichnen, Y Wasserstoff, C₁- bis C₁₂-Alkyl, Phenyl oder C₁- bis C₄-Alkoxycarbonyl bedeutet und k für die Zahl 1, 2 oder 3 sowie m und n jeweils für Zahlen von 0 bis 50 stehen, wobei die Summe aus m + n mindestens 4 betragen muß, Phenylalkylgruppen mit 1 bis 20 C-Atomen im Alkyl, Phenyl, einen fünf- oder sechsgliedrigen ungesättigten oder gesättigten heterocyclischen Ring mit bis zu drei Heteroatomen aus der Gruppe Stickstoff, Sauerstoff und Schwefel, welcher zusätzlich benzanelliert sein kann, wobei alle bei den Bedeutungen für R genannten Phenylkerne und heterocyclischen Ringe noch zusätzlich als Substituenten bis zu drei C₁- bis C₄-Alkylgruppen, Hydroxylgruppen, Carboxylgruppen, Sulfogruppen oder C₁- bis C₄-Alkoxycarbonylgruppen tragen können, oder einen Rest der Formel 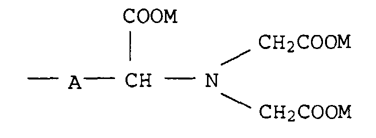 steht, wobei A eine C₁- bis C₁₂-Alkylen-Brücke oder eine chemische Bindung bezeichnet, und
M Wasserstoff, Alkalimetall, Erdalkalimetall, Ammonium oder substituiertes Ammonium in den entsprechenden stöchiometrischen Mengen bedeutet,
durch Umsetzung von entsprechenden 2-substituierten Glycinen oder 2-substituierten Glycinnitrilen oder verdoppelten Glycinen der Formel 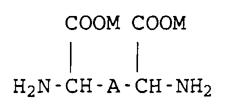 oder verdoppelten Glycinnitrilen der Formel 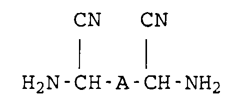 oder von Alaninaminonitril oder 5-Methylhydantoin im Falle von Alanin als Vorstufen der als Ausgangsmaterial genannten Glycinderivate mit Formaldehyd und Alkalimetallcyanid in wäßrigem Medium bei einem pH-Wert von 8 bis 14, dadurch gekennzeichnet, daß man das Ausgangsmaterial im wäßrigen Reaktionsmedium auf Umsetzungstemperatur bringt, danach 0,5 bis 30 % der zur Umsetzung benötigten Menge an Alkalimetallcyanid zu den vorgelegten Glycinderivaten oder deren Vorstufen gibt und anschließend die restliche Menge Alkalimetallcyanid und den Formaldehyd gleichzeitig über einen Zeitraum von 0,5 bis 12 Stunden zudosiert.

2. Verfahren zur Herstellung von Glycin-N,N-diessigsäure-Derivaten I nach Anspruch 1, dadurch gekennzeichnet, daß man vor und/oder während der Umsetzung ein Vakuum von 10 bis 100 kPa (100 bis 1000 mbar) an die Reaktionsapparatur anlegt.

3. Verfahren zur Herstellung von Glycin-N,N-diessigsäure-Derivaten I nach Anspruch 1 oder 2, dadurch gekennzeichnet, daß man vor und/oder während der Umsetzung ein Inertgas durch die Reaktionsmischung bzw. die vorgelegten Umsetzungskomponenten leitet.

4. Verfahren zur Herstellung von Glycin-N,N-diessigsäure-Derivaten I nach den Ansprüchen 1 bis 3, dadurch gekennzeichnet, daß man als Ausgangsmaterial aus der technischen Synthese von Glycinderivaten oder Alaninaminonitril oder 5-Methylhydantoin im Falle von Alanin als deren Vorstufen stammendes nicht gereinigtes Rohmaterial oder bei solchen Synthesen anfallende Mutterlaugen einsetzt.

5. Verfahren zur Herstellung von Glycin-N,N-diessigsäure-Derivaten I nach den Ansprüchen 1 bis 4, bei denen R für C₁- bis C₂₀-Alkyl, C₂- bis C₂₀-Alkenyl oder einen Rest der Formel 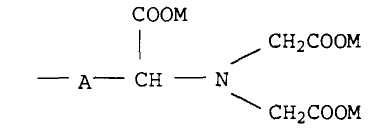 steht.

6. Verfahren zur Herstellung von α-Alanin-N,N-diessigsäure und ihren Alkalimetall-, Ammonium- und substituierten Ammoniumsalzen nach den Ansprüchen 1 bis 4.

## Claims

1. A process for preparing glycine-N,N-diacetic acid derivatives of the general formula I 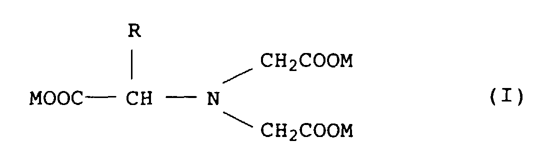 where
R is C₁-C₃₀-alkyl or C₂-C₃₀-alkenyl, each of which can additionally carry as substituents up to 5 hydroxyl groups, formyl groups, C₁-C₄-alkoxy groups, phenoxy groups or C₁-C₄-alkoxycarbonyl groups and can be interrupted by up to 5 nonadjacent oxygen atoms, or alkoxylate groups of the formula -(CH₂)ₖ-O-(A¹O)ₘ-(A²O)ₙ-Y, where A¹ and A² are, independently of one another, 1,2-alkylene groups having 2 to 4 carbon atoms, Y is hydrogen, C₁-C₁₂-alkyl, phenyl or C₁-C₄-alkoxycarbonyl, and k is 1, 2 or 3 and m and n are each from 0 to 50, it being necessary for the total of m + n to be at least 4, phenylalkyl groups having 1 to 20 carbon atoms in the alkyl, phenyl, a five- or six-membered unsaturated or saturated heterocyclic ring which has up to three hetero atoms from the group consisting of nitrogen, oxygen and sulfur and which can additionally be benzo-fused, it also being possible for all the phenyl nuclei and heterocyclic rings mentioned in the meanings for R additionally to carry as substituents up to three C₁-C₄-alkyl groups, hydroxyl groups, carboxyl groups, sulfo groups or C₁-C₄-alkoxycarbonyl groups, or a radical of the formula 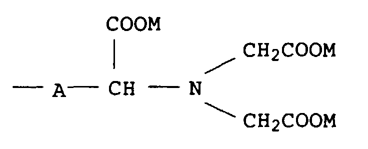 where A is a C₁-C₁₂-alkylene bridge or a chemical bond, and
M is hydrogen, alkali metal, alkaline earth metal, ammonium or substituted ammonium in the appropriate stoichiometric amounts,
by reacting corresponding 2-substituted glycines or 2-substituted glycinonitriles or doubled glycines of the formula 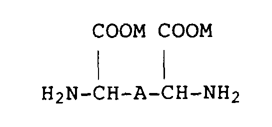 or doubled glycinonitriles of the formula 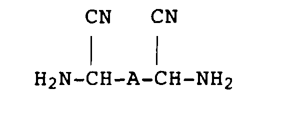 or alanine amino nitrile or 5-methylhydantoin in the case of alanine as precursors of the glycine derivatives named as starting material with formaldehyde and alkali metal cyanide in aqueous medium at a pH of from 8 to 14, wherein the starting material is brought to reaction temperature in the aqueous reaction medium, then from 0.5 to 30% of the amount of alkali metal cyanide required for the reaction are added to the glycine derivatives or precursors thereof, and subsequently the remaining amount of alkali metal cyanide and the formaldehyde are simultaneously metered in over a period of from 0.5 to 12 hours.

2. A process for preparing glycine-N,N-diacetic acid derivatives I as claimed in claim 1, wherein the pressure in the reaction apparatus is from 10 to 100 kPa (100 to 1000 mbar) before and/or during the reaction.

3. A process for preparing glycine-N,N-diacetic acid derivatives I as claimed in claim 1 or 2, wherein an inert gas is passed through the reaction mixture or the reactants initially present before and/or during the reaction.

4. A process for preparing glycine-N,N-diacetic acid derivatives I as claimed in any of claims 1 to 3, wherein unpurified raw material derived from the industrial synthesis of glycine derivatives or alanine amino nitrile or 5-methylhydantoin in the case of alanine as their precursors, or mother liquors produced in such syntheses, is used as starting material.

5. A process for preparing glycine-N,N-diacetic acid derivatives I as claimed in any of claims 1 to 4, where R is C₁-C₂₀-alkyl, C₂-C₂₀-alkenyl or a radical of the formula

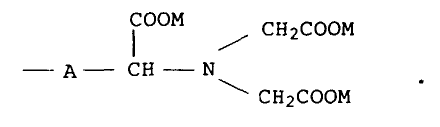

6. A process for preparing α-alanine-N,N-diacetic acid and its alkali metal, ammonium and substituted ammonium salts as claimed in any of claims 1 to 4.

## Revendications

1. Procédé de préparation de dérivés d'acide glycine-N,N-diacétique de formule générale I 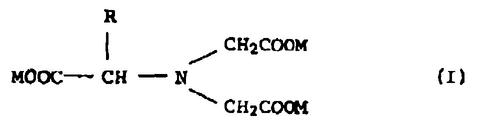 dans laquelle
R est mis pour un groupe alkyle en C₁ à C₃₀ ou alcényle en C₂ à C₃₀, lesquels portent en plus comme substituants jusqu'à 5 groupes hydroxyle, groupes formyle, groupes alcoxy en C₁ à C₄, groupes phénoxy ou groupes (alcoxy en C₁ à C₄)-carbonyle et peuvent être interrompus par jusqu'à 5 atomes d'oxygène non voisins, des groupements alcoxylate de formule - (CH₂)ₖ-O-(A¹O)ₘ- (A²O)ₙ-Y, dans laquelle A¹ et A² désignent indépendamment l'un de l'autre des groupes 1,2-alkylène ayant 2 à 4 atomes de C, Y représente un atome d'hydrogène, un groupe alkyle en C₁ à C₁₂, phényle ou (alcoxy en C₁ à C₄)-carbonyle et k est mis pour le nombre 1, 2 ou 3 et, m et n sont mis respectivement pour des nombres de 0 à 50, la somme de m + n devant s'élever à au moins 4, des groupes phénylalkyle ayant 1 à 20 atomes de C dans le groupe alkyle, phényle, un hétérocycle insaturé ou saturé à cinq ou six maillons ayant jusqu'à trois hétéroatomes choisis dans le groupe des atomes d'azote, d'oxygène et de soufre, lequel peut être de plus benzocondensé, tous les hétérocycles et noyaux phényle cités pour les significations de R pouvant encore porter de plus comme substituants jusqu'à trois groupes alkyle en C₁ à C₄, des groupes hydroxyle, des groupes carboxyle, des groupes sulfo ou des groupes (alcoxy en C₁ à C₄)-carbonyle, ou bien pour un résidu de formule 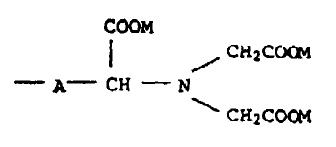 dans laquelle A désigne un pont alkylène en C₁ à C₁₂ ou une liaison chimique, et
M représente un atome d'hydrogène, un métal alcalin, un métal alcalino-terreux, un groupe ammonium ou ammonium substitué dans les quantités stoechiométriques correspondantes,
au moyen d'une réaction de glycines 2-substituées ou des glycine-nitriles 2-substitués correspondants ou de glycines doubles de formule 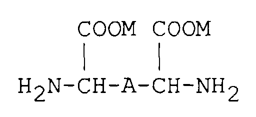 ou de glycine-nitriles doubles de formule 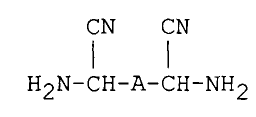 ou d'alanine-aminonitrile ou de 5-méthylhydantoïne dans le cas de l'alanine comme précurseurs des dérivés de glycine cités comme matière de départ, avec du formaldéhyde et un cyanure de métal alcalin dans un milieu aqueux à une valeur de pH de 8 à 14, caractérisé en ce que l'on amène la matière de départ dans le milieu réactionnel aqueux à une température de réaction, qu'ensuite, on ajoute 0,5 à 30% de la quantité nécessaire à la réaction, de cyanure de métal alcalin aux dérivés de glycine ou à leurs précurseurs présents puis on ajoute simultanément par doses la quantité restante de cyanure de métal alcalin et le formaldéhyde pendant une durée de 0,5 à 12 heures.

2. Procédé de préparation de dérivés de l'acide glycine-N,N-diacétique I selon la revendication 1, caractérisé en ce que, l'on fait un vide de 10 à 100 kPa (100 à 1 000 mbars) dans l'appareil de réaction, avant et/ou pendant la réaction.

3. Procédé de préparation de dérivés d'acide glycine-N,N-diacétique I selon la revendication 1 ou 2, caractérisé en ce que l'on fait passer un gaz inerte à travers le mélange réactionnel ou bien les composants de réaction présents avant et/ou pendant la réaction.

4. Procédé de préparation de dérivés de l'acide glycine-N,N-diacétique I selon les revendications 1 à 3, caractérisé en ce que l'on met en oeuvre comme matière de départ, une matière première non purifiée provenant de la synthèse industrielle des dérivés de glycine ou de l'alanine-aminonitrile ou de la 5-méthylhydantoïne dans le cas de l'alanine en tant que précurseurs ou des liqueurs mères produites dans de telles synthèses.

5. Procédé de préparation de dérivés d'acide glycine-N,N-diacétique I selon les revendications 1 à 4, dans lesquels R est mis pour un groupe alkyle en C₁ à C₂₀, alcényle en C₂ à C₂₀ ou un résidu de formule

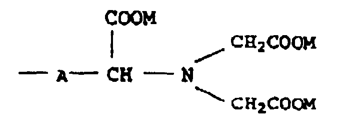

6. Procédé de préparation de l'acide α-alanine-N,N-diacétique et de ses sels de métal alcalin, d'ammonium et d'ammonium substitué selon les revendications 1 à 4.
